(19) **Europäisches Patentamt European Patent Office Office européen des brevets**

(11) **EP 4 481 031 A1**

(12) **EUROPEAN PATENT APPLICATION**

(43) Date of publication:
25.12.2024 Bulletin 2024/52

(21) Application number: 23191755.0

(22) Date of filing: 16.08.2023

(51) International Patent Classification (IPC):
*C12M 1/34* (2006.01)        *C12M 1/36* (2006.01)
*C12N 1/20* (2006.01)

(52) Cooperative Patent Classification (CPC):
**C12M 41/46; C12M 41/48; C12N 1/20;**
C12R 2001/19

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: 22.06.2023 LT 2023528

(71) Applicant: Kaunas University of Technology
44029 Kaunas (LT)

(72) Inventors:
• **Urniezius, Renaldas**
  53331 Kaunas (LT)
• **Survyla, Arnas**
  53368 Miriniskiai, Kauno rajonas (LT)
• **Levisauskas, Donatas**
  51390 Kaunas (LT)
• **Simutis, Rimvydas**
  46307 Kaunas (LT)
• **Masaitis, Deividas**
  60186 Raseiniai (LT)

(74) Representative: **Klimaitiene, Otilija**
**AAA Law**
**A. Gostauto 40B**
**03163 Vilnius (LT)**

(54) **METHOD OF TARGET PRODUCT SYNTHESIS MAXIMIZATION IN SEMI-BATCH CULTIVATION PROCESSES OF RECOMBINANT E.COLI**

(57) The invention discloses a method to maximize the synthesis and concentration of a target-product in semi-periodic cultivation processes of recombinant *Ecoli* by controlling the process in the post-induction period according to an optimized specific bacterial growth-rate profile. This method is based on experimental studies of the cultivation of *E.coli* bacteria, which found that the maximum concentration of the synthesized target product at the end of cultivation is determined by the concentration of *E.coli* biomass, the average age of the population at the time of process induction, and the optimal control of the bacterial specific growth rate after the induction moment. Biomass concentration and population mean age are derived from indirect measurement methods in real time. During induction, an empirical expression determines the maximum possible concentration of the target product at the end of the process. An accumulation profile of the target product is then created, in the formation of which the average age of the bacterial population, which depends on the bacterial specific growth-rate, is a key factor. Based on real-time optimization procedures, an optimal specific growth rate profile is determined for the post-induction cultivation period that maximizes the concentration of the target product at the end of the cultivation process.

1. Evaluation of biomass concentration x(t) and average age of bacteria Age(t) during E.coli cultivation process

2. Determination of the expected maximum concentration of the target product at the end of cultivation, at the moment of induction, $t_{induction}$

3. An iterative procedure for target product maximization: optimization of a specific growth profile

4. Use of the specific growth rate profile in a bioreactor automatic controller to adjust the nutrient substrate flow

Bioreactor automatic controller

Bioreactor and E. coli cultivation process therein

Nutrient substrate flow

Fig. 1

EP 4 481 031 A1

**Description**

FIELD OF THE INVENTION

The invention is related to technologies of automatic control of bioreactors, more specifically, to a method of automatic control that enables maximizing the yield of synthesis of the target product in semi-batch cultivation processes of recombinant *Ecoli.* The aforementioned product yield maximization is implemented by forming optimized *E.coli* bacteria specific growth profiles in real time and using them to design the *E.coli* cultivation process in the post-induction period.

TECHNICAL LEVEL

*E.coli* bacteria are well-studied, exhibit high growth rates, and their recombinant versions can synthesize a variety of target proteins (i.e. cultivation target products). Therefore, cultures of *E.coli* bacteria are often cultivated with the aim of producing recombinant proteins in industrial bioreactors. Currently, the main part of recombinant proteins used for biomedical purposes is obtained in the cultivation processes of recombinant E.coli bacteria, which are carried out in modern bioreactors with a high level of automation. *E.coli* cultivation processes for target products are carried out in two stages:

a) in the first stage (pre-induction period), a high growth rate of *E.coli* bacteria is maintained and a high concentration of *E.coli* biomass is achieved in the bioreactor;
b) in the second stage (post-induction period), the synthesis of the target product is initiated with the help of a special chemical inducer, which, by regulating the flow of the nutrient substrate fed to the bioreactor together with the growth rate of E.coli bacteria, is maintained until the end of the cultivation process.

In these recombinant *E.coli* cultivation processes, the nutrient medium (nutritional substrate) is supplied to the bacteria gradually during the entire cultivation process. Such a gradual supply allows to control the concentration of the substrate in the nutrient medium in the bioreactor, thus avoiding excessively high concentrations of the substrate and excessive formation of by-products of metabolism, which would inhibit the growth of bacteria and the synthesis of the target product. In the cultivation processes of recombinant bacteria, the synthesis of the target product also depends significantly on the average age of the bacterial population. Younger cultures of *E.coli* bacteria in logarithmic growth phase typically synthesize more of the target product than older cultures in stationary growth phase. However, the relationship between bacterial age and target product synthesis efficiency (synthesis rate) also depends on the type of bacteria and their cultivation conditions. Therefore, this relationship in a specific cultivation process would be rationally identified based on the data of a real cultivation experiment. After identifying the interdependence between the average age of the bacterial population and the synthesis rate of the target product, it is possible to use this dependence to more effectively control the flow of the feeding substrate and maximize the yield (product concentration) of the target product in the post-induction cultivation period. This interdependence was exploited in the present invention, enabling the development and implementation of a method for maximizing the synthesis of the target product.

Various methods and technical solutions are known for regulating the flow of nutrient substrate supplied to the bioreactor, which aim to more efficiently manage *E.coli* cultivation processes and achieve high productivity.

US patent US5595905A, 1997, *Process Control System for Fed-Batch Fermentation Using a Computer to Predict Nutrient Consumption* **[1]** describes the regulation system for supplying the nutrient substrate to the bioreactor, which is intended to control semi-batch cultivation processes of microorganisms. This system is based on laboratory experiments of nutrient substrate concentration measurements and calculation procedures that allow the calculation of the nutrient substrate flow for the following stage of the cultivation experiment. However, the average age of the bacterial population is not taken into account in this system running the bacteria feeding algorithm. Therefore, the system is inefficient if the synthesis phase of the target product requires the use of feeding substrate at a rate lower than the maximum. Also, this system is problematic to implement in industrial bioreactors and cultivation processes because it requires a lot of maintenance costs due to frequent experimental measurements that must be performed in the laboratory.

Another US patent US6284453B1, 2001, *Method for Controlling Fermentation Growth and Metabolism* **[2]** discloses a method of supplying a nutrient substrate flow to a bioreactor, where the substrate flow is adjusted to maintain a given growth rate of *E.coli* bacteria, which is determined by a series of direct and indirect measurements. This method requires predicting the growth rate of bacteria before the start of cultivation and is not suitable for maximizing the target product, because it is difficult to predict before the start of the cultivation process the specific growth rate of bacteria required to maintain the specific growth rate in the post-induction period, which would maximize the concentration of the target product at the end of the cultivation process. Also, when implementing this method, it is necessary to carry out frequent laboratory measurements of essential bioprocess variables, so the application of this method in industrial biotechnological processes is relatively difficult.

Lithuanian patent LT6395B [3] discloses an indirect method for regulating the nutrient substrate, in which the flow of the nutrient substrate supplied to the bioreactor is changed in such a way as to ensure a predetermined specific growth rate of microorganisms, which is determined indirectly by measuring the rate and amount of oxygen consumption of the culture grown in the bioreactor. However, the application of this method for maximizing the yield of the target product is not effective, because the average age of the bacterial population is not taken into account when forming the specific growth rate of microorganisms determined by the bioprocess operator, and at the same time, the appropriate nutrient substrate supply mode for the maximization of the target product during the period of the post-induction cultivation process is not ensured.

The closest prior art to this invention is the method of regulating the nutrient substrate flow to control the semi-batch cultivation process of recombinant *E.coli,* described in [4] (Gnoth, Stefan; Simutis, Rimvydas; Lübbert, Andreas. Selective Expression of the Soluble Product Fraction in Escherichia Coli Cultures Employed in Recombinant Protein Production Processes // Applied Microbiology and Biotechnology. New York: Springer. ISSN 0175-7598. 2010, Vol. 87, iss. 6, p. 2047-2058)0. In this method, the profile of nutrient substrate supply during the post-induction period is selected to support the specific growth profile of bacteria formed by the hybrid model, which enables high concentration of the synthesized target product at the end of cultivation. The main drawback of this method: in order to form feeding profiles of the post-induction period, it is necessary to create hybrid mathematical models (defined by a combination of fundamental models and artificial neural network models) that would adequately describe the cultivation processes of recombinant *E.coli* bacteria. The preparation of such models requires a lot of labor and time, so the application of this method in industrial *E.coli* cultivation processes is very limited.

SUMMARY OF THE INVENTION

**Technical problem.** This invention solves the technical problem - increasing the efficiency of the synthesis of the target product in the semi-batch cultivation processes of recombinant *E.coli.* I.e. with lower costs of cultivation process and bioreactor management (including process design, process operator workload and error probability), a higher yield of the synthesized target product may be achieved.

**Solution.** The present invention discloses a method for controlling the *E.coli* cultivation process and maximizing the yield of target product synthesis, which avoids the disadvantages of the above control solutions. The method is implemented on the basis of empirical mathematical models formed on the basis of cultivation experiment data and a computerized numerical optimization procedure. This procedure makes it possible to form an optimized profile of the specific growth rate of *E.coli* bacteria in real time during the post-induction cultivation period, which is further used in the automatic controller of the bioreactor processes, to control the flow of the nutrient substrate fed to the bioreactor. The control and maximization method involves the following essential steps:

1) In the first step, the estimation of the *E.coli* biomass concentration and the calculation of the average age of the bacterial population during the *E.coli* cultivation period before induction ($t < t_{induction}$) are performed. In this step, the operator of the biotechnological process, depending on the growth of the biomass concentration and the changing characteristics of the technological processes of the bioreactor supporting it (the number of revolutions of the stirrer, the flows of supplied air and supplied oxygen), controls the flow of the feeding substrate, which leads to a high growth rate of recombinant *Ecoli*. Assessment of biomass concentration $x(t)$ is performed in real time according to a known method that measures biomass concentration indirectly, according to oxygen consumption rate indicators [5] (Survyla, A., Urniezius, R. & Simutis, R. Viable Cell Estimation of Mammalian Cells Using Off-Gas-Based Oxygen Uptake Rate and Aging-Specific Functional. Talanta 254, 124121, 2023).

$$\begin{cases} x(t_i) = x_0 + \sum_{j=1}^{i} \frac{OUR(t_j)}{\alpha(t_j)} \Delta t_j; & \text{if } \sum_{j=1}^{i-1} X(t_j)\Delta t_j \leq k_{cX} \\ x(t_i) = \dfrac{x_0 + \sum_{j=1}^{i} \frac{OUR(t_j)+\beta(t_j)\cdot X_{cX}}{\alpha(t_j)} e^{\sum_{k=1}^{j} \frac{\beta(t_k)}{\alpha(t_k)}\Delta t_k} \Delta t_j}{e^{\sum_{j=1}^{i} \frac{\beta(t_j)}{\alpha(t_j)}\Delta t_j}}, & \text{otherwise,} \end{cases} \quad (1)$$

where x represents the biomass concentration [g/L], *OUR* represents the oxygen consumption rate [g/(h*L)], $x_0$ represents the biomass concentration at the moment of inoculation, $\alpha$ and $\beta$ represent the stoichiometry coefficients of the culture, $k_{cX}$ represents the parameter that defines the start $t_{cX}$ from which the biomass support member $\beta$ begins to participate in expression (1) and $x_{cX}$ represents the value of the biomass concentration at that time ($k_{cX} \equiv$

$\sum_{j=1}^{t_{cX}} X(t_j)\Delta t_j, x_{cX} \equiv x(t_{cX})$). After estimating the biomass concentration according to equation (1), the average age of the population of *E.coli* bacteria *Age(t)* is further estimated, which can be calculated according to the following formula:

$$Age(t_i) = \frac{t_i \cdot x(t_i) - \sum_{j=1}^{i} t_j \cdot \Delta x_{j,j-1}}{x(t_i)} \qquad (2)$$

**2)** In the second step, based on expression (1) the estimated biomass concentration at the time of induction $x(t_{induction})$ and the average age of the bacterial population $Age(t_{induction})$ estimated from it, and after applying the interdependence identified on the basis of data from previous *E.coli* cultivation experiments, the probable maximum concentration $P_{max}$ of the target product at the end of the cultivation process is estimated. The formula for this dependency is as follows:

$$P_{max} = \frac{x(t_{induction})}{Age(t_{induction})^2} * Y_{ap} \ [g/L]; \qquad (3),$$

where $Y_{ap}$ represents a yield factor for a specific *E.coli* cultivation process based on the data from previous experiments, which characterizes the capacity of those species of bacteria to synthesize the target protein.

**3)** In the third step, using the identified expected maximum concentration $P_{max}$ of the target product, the target product concentration function $P(t)$ in the post-induction cultivation period $t=[t_{induction}; t_{end}]$ is calculated using expression (4) below. In the formula of the product concentration function $P(t)$, the main arguments are the probable maximum concentration $P_{max}$ and the average age of the bacterial population $Age(t)$, where $P(t_{induction}) = 0$:

$$P(t_i) = P(t_{i-1}) + \frac{P_{max} - P(t_{i-1})}{Age(t_i)} \Delta t_i \ , \ t_i = [t_{induction}; t_{end}] \qquad (4)$$

In the present invention, the specific bacterial growth rate $\mu(t)$ is selected and generated using an iterative optimization procedure. During this iterative procedure, such a $\mu$-profile of bacterial specific growth $\mu(t)$, $t=[t_{induction}; t_{end}]$ is selected and formed through a series of iterations, which would enable seeking the predicted maximum product concentration $P(t_{end})=P_{max}$ at the end of cultivation.

**4)** In the fourth step of the method, according to the formed specific growth rate $\mu$-profile $\mu(t)$, $t=[t_{induction}; t_{end}]$, the automatic controller of the bioreactor controls the flow of the nutrient substrate (for example, using the specific growth rate control system proposed by [6] (Galvanauskas, Vytautas; Simutis, Rimvydas; Levišauskas, Donatas; Urniežius, Renaldas. Practical Solutions for Specific Growth Rate Control Systems in Industrial Bioreactors // Processes. Basel: MDPI. ISSN 2227-9717. 2019, vol. 7, iss. 10, art. No. 693, p. 1-13.), thereby maintaining the specific growth rate $\mu$ defined by the $\mu$-profile in the bioreactor.

**Novelty.** The new features of this method, in individual steps and as a whole, are as follows:

- in the first step, a new indicator of the quality of cultivation and synthesis is evaluated - the average age of the bacterial population *Age(t)*, which partially determines the capacity of the *E.coli* culture to synthesize the target product (protein) in the post-induction period;
- in the second step, based on the new formulas created, the expected maximum concentration of the target product $P_{max}$ is estimated, as well as the expected concentration growth function $P(t)$ in the post-induction period $t=[t_{induction}; t_{end}]$;
- in the third step, applying the method of iterative optimization and a new expression for the accumulation of the target product based on the data of cultivation experiments, a $\mu$-profile of the specific growth rate is formed, the application of which in the cultivation process predicts the likely maximum concentration of the target product at the end of the cultivation process.

**Technical effects.** The invention has the following technical effects:

- A higher relative yield of the target product is achieved. The analysis of the experimental data showed that in the process of cultivating recombinant *E.coli,* by synthesizing the target product in this way, the yield of the target product synthesis is obtained on average -12% higher than when using known methods of nutrient substrate management;

- The automated control of the cultivation process using empirical *E.coli* cultivation models obtained experimentally is simpler and more reliable. A more easily controlled automated process, accordingly, does not require a skilled operator and his frequent involvement in adjusting the nutrient substrate flow;
- This way of controlling and maximizing product synthesis can be effectively applied in the industrial *E.coli* cultivation processes and bioreactors.

DESCRIPTION OF DRAWINGS

The features and advantages of the invention are described in the detailed description of the invention with reference to the drawings below:

**Fig. 1** shows steps (1.1, 1.2, 1.3, 1.4) of the technological method for maximizing the concentration of the synthesized target product in semi-batch cultivation processes of recombinant *E.coli* (5): 6 - estimation (2) of the biomass concentration *x(t)* in the bioreactor (5) in real time; 3 - bioreactor automatic controller; 4 - *E.coli* cultivation system environment: 5 - bioreactor, and 3 - automatic controller; 6 - *E.coli* nutrient substrate flow.

**Fig. 2** shows the diagram of the *E.coli* cultivation process control system, applying the method for control and product synthesis maximization disclosed in the invention.

**Fig. 3** Comparisons of the results of *E.coli* cultivation experiments after optimizing the growth of the target product concentration after induction. Represented graph of:

**a)** Biomass concentration *x(t),*
**b)** The average age of bacterial population *Age(t),*
**c)** Specific growth rate $\mu(t),$ and
**d)** Variation of target product concentration *P(t)* in a typical *E.coli* cultivation experiment, which demonstrates what the difference between the experimental results and the predicted results would be if the target product synthesis process was controlled according to the optimized specific growth rate profile $\mu(t)$ ($\mu$-profile).

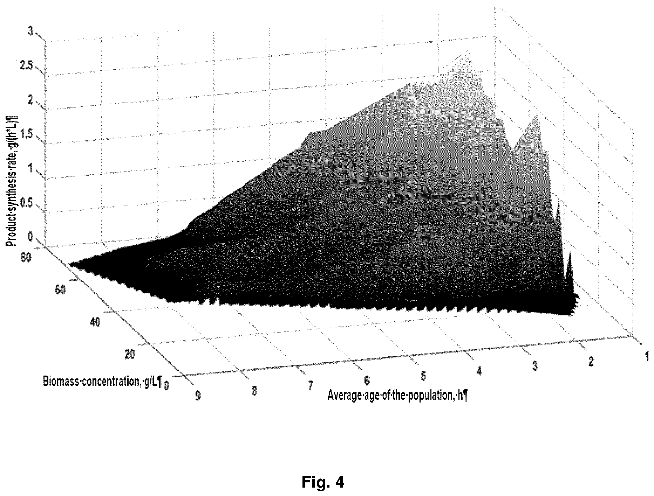

**Fig. 4** 3D-graph is shown: the dependence of the target product synthesis (product concentration growth) rate $dP(t)/dt$ on the variables of *E.coli* biomass concentration *x(t)* and the population average age *Age(t).*

LIST OF TERMS

- **The average age of a bacterial population** (*Age*) means the average life time of a bacterium, from the time the bacterium divides to the moment of analysis *(t).* Basically, it depends on the specific growth rate of the bacteria $\mu$;
- **Specific bacterial growth rate** ($\mu$) means the change in bacterial concentration calculated for the total bacterial concentration *x,* $\mu = dx/(dt\,x)$, [1/h];
- **Biomass/bacteria concentration** (*x*) means the concentration of bacteria dried according to the standard protocol in the culture medium [g/L];
- **Induction** means a technological step during which a chemical component is dosed into the bioreactor, usually IPTG (*Isopropyl $\beta$-D-1-thiogalactopyranoside*), which initiates the synthesis of the target product in recombinant *E.coli;*
- **Induction moment** means time mark when induction is performed (hours);
- **Target product** means the specific proteins synthesized during the cultivation of recombinant *E.coli* bacteria, such as insulin, interferon, growth factors, trypsin, and others, which are used in medicine. *E.coli (Escherichia coli)* is one of the most suitable bacterial species for synthesizing recombinant proteins. It is the most widely used expression platform (vessel) as a bacterial factory to create heterologous proteins, including a large collection of expression plasmids. Bacteria can produce/secrete unwanted metabolic products when not properly cultivated. The present invention focuses on a target product that is usually synthesized within the bacterium, directly from the carbon source, without extraneous metabolic pathways. Recombinant *E.coli* bacteria are genetically modified according to the selected genome, then the modified bacteria produce the corresponding target product: sfGFP-proteins, interferon, trypsin, and others.

DETAILED DESCRIPTION OF THE INVENTION

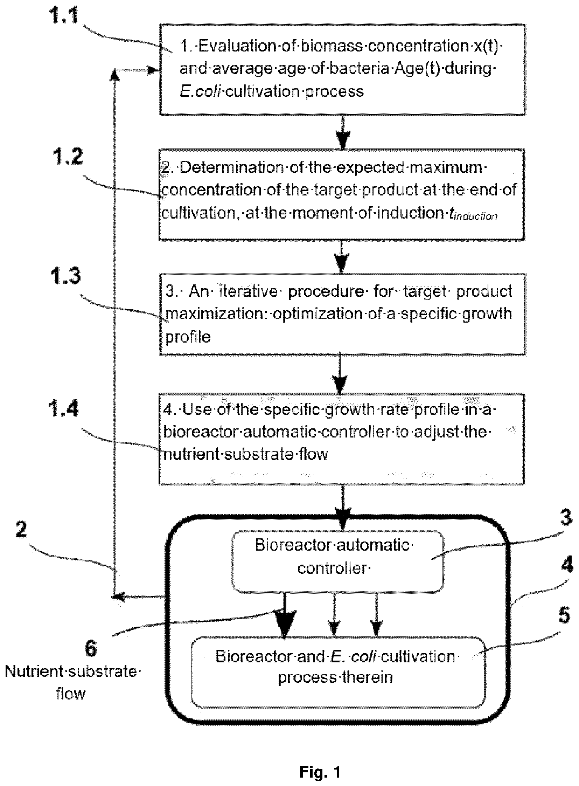

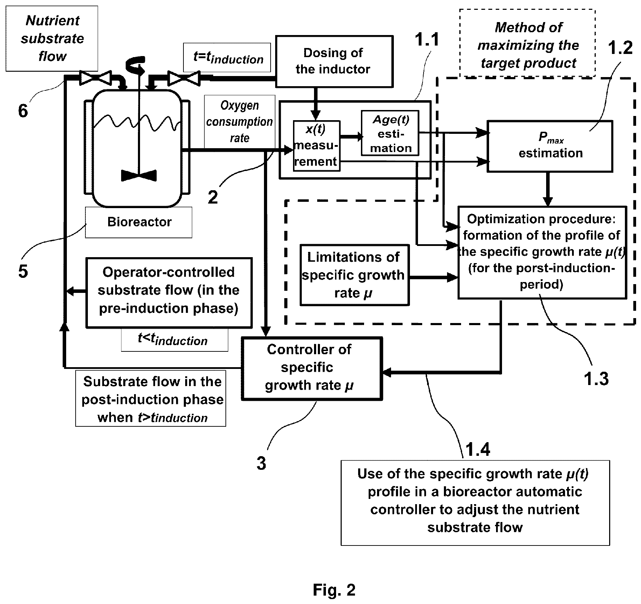

The steps of the technological process to maximize the concentration of the target product synthesized by recombinant *E.coli* in semi-periodic cultivation processes, planning a specific bacterial growth rate profile in the post-induction period, are shown in Fig. 1. This implementation of the method in the *E.coli* cultivation process, bioreactor and its control system with essential functional elements is shown in Fig. 2. The implementation details of each step are detailed below.

In the first step of the method, during the cultivation of *E.coli* bacteria, the estimation of the biomass concentration $x(t)$ ((expression 1)) and the average age of the bacterial population *Age(t)* ((expression 2)) are performed in real time, by means of indirect measurements in the bioreactor. This step is carried out during the period of cultivation of *E.coli* bacteria until the moment of initiation of induction ($t_{induction}$). In this (pre-induction) period, efforts are made to maintain a high rate of bacterial growth, so the nutrient substrate is supplied to the bioreactor in such a way that the concentration of the substrate in the bioreactor medium would limit bacterial growth as little as possible. For typical cultivation processes of recombinant *Ecoli,* the pre-induction cultivation period continues as long as the technical characteristics of the bioreactor still allow maintaining an acceptable concentration of dissolved oxygen in the cultivation medium. In this step (i.e. in the pre-induction period), the operator of the biotechnological process, depending on the characteristics of the biomass growth support processes (number of revolutions of the mixer, supplied air and supplied oxygen flows), plans and controls the flow of the nutrient substrate, which would result in a high growth rate of recombinant *E.coli*. Within the scope of the present invention, the flow of the nutrient substrate planned by the operator and its control up to the moment of induction are carried out normally without special changes and improvements.

The real-time estimation of biomass concentration $x(t)$, in this invention, can be carried out by a known indirect measurement method, according to the measured indicators of the rate of oxygen used during biosynthesis [5] (Survyla, A., Urniezius, R. & Simutis, R. Viable Cell Estimation of Mammalian Cells Using Off-Gas-Based Oxygen Uptake Rate and Aging-Specific Functional. Talanta 254, 124121, 2023).

$$\begin{cases} x(t_i) = x_0 + \sum_{j=1}^{i} \frac{OUR(t_j)}{\alpha(t_j)}\Delta t_j; & if \ \sum_{j=1}^{i-1} X(t_j)\Delta t_j \leq k_{cX} \\ \\ x(t_i) = \dfrac{x_0 + \sum_{j=1}^{i} \frac{OUR(t_j)+\beta(t_j)\cdot X_{cX}}{\alpha(t_j)} e^{\sum_{k=1}^{j} \frac{\beta(t_k)}{\alpha(t_k)}\Delta t_k} \Delta t_j;}{e^{\sum_{j=1}^{i} \frac{\beta(t_j)}{\alpha(t_j)}\Delta t_j}}, & otherwise, \end{cases} \quad (1)$$

where $x$ represents the biomass concentration [g/L], *OUR* is the oxygen consumption rate [g/(h*L)], $x_0$ represents the biomass concentration at the moment of inoculation, $\alpha$ and $\beta$ represent the stoichiometry coefficients of the culture, $k_{cX}$ represents the parameter that defines the start from which the biomass support member $\beta$ begins to participate in expression (1) and $x_{cX}$ represents the value of the biomass concentration at that time $(k_{cX} \equiv \sum_{j=1}^{t_{cX}} X(t_j)\Delta t_j, x_{cX} \equiv x(t_{cX}))$.

One of the essential aspects of this invention is that the average age of the bacterial population *Age(t)* is taken into account when maximizing the synthesis of the target product. The parameter *Age(t)* depends on how fast bacteria grow and divide. To estimate *Age(t)* values, the following mathematical expression (2) is used:

$$Age(t_i) = \frac{t_i \cdot x(t_i) - \sum_{j=1}^{i} t_j \cdot \Delta x_{j,j-1}}{x(t_i)}. \quad (2)$$

In the second step of the method, the expected maximum concentration $P_{max}$ of the target product to be synthesized at the end of the cultivation process at the moment $t_{end}$ is estimated, which depends on the biomass concentration $x(t_{induction})$ and the average age of the population $Age(t_{induction})$ estimated at the moment of induction. After analyzing the data of the cultivation experiments, it was observed that at the end of the cultivation process, the highest concentration of the target product $P(t_{end})$ was obtained in those cultivation experiments where the average age of *E.coli* at the moment of induction $Age(t_{induction})$ was the lowest and the biomass concentration $x(ti_{nduction})$ was the highest. This complex dependence is shown in Fig. 4 as a 3D function of the two variables $x(t)$ and *Age(t),* showing the dependence of the rate of product synthesis on these two factors. Based on these results of cultivation experiments, a formula was developed to estimate the expected maximum target product concentration $P_{max}$ at the end of the cultivation process (i.e. $P(t_{end})$)

$$P_{max} = \frac{x(t_{induction})}{Age(t_{induction})^2} * Y_{ap} \ [g/L]; \quad (3)$$

where the multiplier $Y_{ap}$ represents a yield factor that characterizes the capacity of *E.coli* cultured in this process to synthesize the target protein selected for a specific cultivation process according to its experimental data (for example, as shown in Fig. 4).

This empirically based formula was obtained by processing a large amount of data from cultivation experiments collected during semi-batch cultivation processes of recombinant E.coli. For example, the following - the results of which have already been published in the scientific press: **[7]** (Survyla, A., Levisauskas, D., Urniezius, R. & Simutis, R. An Oxygen-Uptake-Rate-Based Estimator of the Specific Growth Rate in Escherichia Coli BI21 Strains Cultivation Processes. Computational and Structural Biotechnology Journal 19, 5856-5863 (2021), **[8]**; Schaepe, S., Kuprijanov, A., Simutis, R. & Lübbert, A. Avoiding Overfeeding in High Cell Density Fed-Batch Cultures of E. Coli During the Production of Heterologous Proteins. Journal of Biotechnology 192, 146-153 (2014)). From these cultivation experiments and processes, the yield factor $Y_{ap}$ = 0.5 was identified in formula (3).

Formula (3) characterizes that the expected maximum concentration value of the target product, $P_{max}$, is determined by the course of the *E.coli* cultivation process up to the moment of induction $t_{induction}$. If the induction is carried out at a high biomass concentration $x(t_{induction})$ of *E.coli,* then usually the average age of the bacterial population $Age(t_{induction})$ is already high and the synthesis of the target product slows down in the post-induction period. On the other hand, if the induction is initiated at a low biomass concentration $x(t)$, then the mean age of the population $Age(t)$ is low and the biosynthesis of the target product is intensive, but due to the low biomass concentration $x(t)$, the concentration of the synthesized product $P(t)$ achieved at the end of cultivation is also low. Thus, the proper selection of the induction moment $t_{induction}$ is important for the result of the cultivation process. Currently, in cultivation processes, the moment of induction $t_{induction}$ is usually selected by the process operator based on working practice and analysis of previous cultivation experiments. In the implementation of this invention, it is also deemed that the moment of induction $t_{induction}$ initiation and the flow of the nutrient substrate up to this moment of induction $t_{induction}$ are planned by the process operator. In the experiments of this invention, the induction was initiated at the moment when the maximum allowable revolutions of the bioreactor stirrer and the maximum flow of supplied air are reached to maintain the concentration of dissolved oxygen in the bioreactor medium.

In the third step of the method, the maximum concentration of the target product $P_{max}$ estimated according to formula (3) at the end of cultivation, for the post-induction period of cultivation $t=[t_{induction}; t_{end}]$, the target product concentration accumulation (growth) function $P(t)$ is calculated:

$$P(t_i) = P(t_{i-1}) + \frac{P_{max} - P(t_{i-1})}{Age(t_i)} \Delta t_i; \qquad (4)$$

In this formula (4), the probable maximum concentration of the target product $P_{max}$ and the average age of the bacterial population $Age(t)$ are the main variables.

Initially, all values of the specific growth rate function for the post-induction period $\mu(t)$ are set to zero before the optimization procedure. During the optimization, each time, after the profile of the product concentration function $P(t)$ is calculated (expression (4), $t=[t_{induction}; t_{end}]$, and by varying the values of the specific bacterial growth rate function $\mu(t)$, $t=[t_{induction};t_{end}]$, in the time period $t=[t_{induction}; t_{end}]$ the values of these variables are calculated for each selected time interval

- according to formula (5) - predicted biomass concentration $x(t)$,

$$x(t_i) = x(t_{induction}) \cdot e^{\sum_{k=t_{induction}}^{i} \mu(t_k) \cdot \Delta t_k} \qquad (5)$$

- according to formula (2) - the average age of the bacterial population $Age(t)$,
- according to formula (4) - the concentration of the target product $P(t)$ in the course of post-induction cultivation, that is, in the period $t=[t_{induction}; t_{end}]$.

The concentration of the target product at the end of cultivation $P(t_{end})$ is maximized by an iterative optimization procedure. This iterative procedure, as a result of its implementation, forms an optimized function $\mu(t)$ - $\mu$-profile of the specific growth rate, which would enable *E.coli* to reach the expected maximum product concentration $P(t_{end})$ at the end of cultivation $(t_{end})$.

Iterative optimization procedure. At its beginning, the discretization step d$t$ of time t of the optimized function $\mu(t)$ is selected (in hours (h)), where the size of the discretization step is selected within the limits: 0.1-1 h), and the end time of cultivation $t_{end}$. According to the initiation moment of induction $t_{induction}$ and the end time of cultivation $t_{end}$ - the number of intervals $n$ is counted, where $n=(t_{end} - t_{induction})/$d$t$. In these d$t$ intervals, according to the function $\mu(t)$ optimization procedure, the optimal values of the bacterial specific growth rate $\mu(t)$ in the mentioned intervals will be determined, i.e. $\mu(t_{induction})$, $\mu(t_{induction}+$d$t)$, $\mu(t_{induction}+2$d$t)$, $\mu(t_{induction}+3$d$t)$, ..., $\mu(t_{end}-$d$t)$, $\mu(t_{end})$. Also, at the beginning of the $\mu(t)$

optimization procedure, $P_{max}$ is calculated according to formula (3). After evaluating the values of these parameters, the iterative optimization of the discrete values $\mu(i^*dt),i=0..n$ of the specific growth rate profile function $\mu(t)$ is carried out in the following order:

- For each post-induction period $[t_{induction}; t_{end}]$ for the discrete time interval $i^*dt, i=0..n$ the value of the specific growth rate $\mu(i^*dt), i=0..n$ is increased from the current value (or from 0 at the beginning) by varying it (increasing to 0.8 or decreasing to 0) with quantifier of 0.01 in specific growth rate;
- For each interval $i^*dt$, biomass concentration $x(i^*dt)$ is calculated according to formula (5);
- According to formula (2), the average age of the bacterial population $Age(t)$ during cultivation is calculated, and by expression (4) the product concentration P(ti) for each time interval $i=0..n$ is calculated;
- The product concentration $P(t)$ is checked at the end of the cultivation process, at time $t_{end}$, i.e. in the final ninth interval $n^*dt$. If this value $P(n^*dt)$ is obtained greater than $P(n^*dt)$ calculated in the previous iteration, then this new value of $P(n^*dt)$ is stored together with the values of the specific growth rate profile $\mu(i^*dt),i=0..n$ is incremented/decremented in the current iteration. Then the next optimization iteration $\mu(i^*dt),i=0..n$ is performed, further increasing/decreasing the values of the specific growth rate $\mu(i^*dt),i=0..n$ at the chosen discreteness (as mentioned, for example, 0,01). If the calculated $P(n)$ value in the current iteration is lower than the $P(n)$ value of the previous iteration, then the $\mu(i^*dt)$ discrete value optimization procedure is stopped and the $\mu(i^*dt)$ discrete value of the previous iteration is saved together with the specific growth rate profile $\mu(i^*dt),i=0..n$ because it showed the maximum expected product concentration $P(n)$ at the end of the cultivation (i.e. at the time $t_{end}$ or the ninth time interval).
- In the 4th step of the control method, this optimized profile $\mu(i^*dt),i=0..n$ is used in the specific growth rate controller of the bioreactor, which maintains the set specific growth rate $\mu$ by adjusting the nutrient substrate flow accordingly.

During the entire optimization procedure, in order to ensure the possibilities of practical use of the optimized specific growth rate profile $\mu(i^*dt),i=0..n,$ additional limitations of the maximum allowed specific growth rate are also evaluated in the optimization iterative procedure. In the cultivation process, the maximum specific growth rate $\mu(i^*dt),i=0..n$ depends on the existing biomass concentration $x(t)$, the average age of the bacterial population $Age(t)$ and the target product concentration $P(t)$. Therefore, during the iterations, those values of the specific growth rate $\mu(i^*dt),i=0..n$, are rejected (not used for further maximization of the concentration of the target product), which exceed the value obtained in expression (6). This limitation for *E.coli* cultivation processes is formed by a modified Monod expression:

$$\mu_{Uplimit}(t) = \mu_{max} \cdot \frac{kx}{(kx+x(t))} \cdot \frac{kage}{(kage+Age(t)^2)} \cdot \frac{kp}{(kp+P(t)^2)}; \qquad (6)$$

where $\mu_{max}$ represents the maximum specific growth rate of *E.coli* culture, $kx$ represents biomass inhibition coefficient, $kage$ represents the method age inhibition coefficient, $kp$ represents the product inhibition coefficient. Based on a series of recombinant *E.coli* cultivation experiments, the following values of these parameters were identified: $\mu_{max} = 1.1$ [1/hr], $kx = 80$ [g/L], $kage = 3.5$ [hr], $kp = 20$ [g/L].

In the iterative optimization procedure, the limitation of the maximum synthesis rate $dP(t)/dt$ of the target product is also introduced. The target product concentration $P(t)$ is calculated according to formula (4) at the beginning of the post-induction period. Due to the sensitivity of the formula (4) to the variable $Age(t),$ the values of the product synthesis rate $dP(t)/dt$ may become higher than they are achievable in real cultivation processes and experiments. Therefore, when calculating the synthesis rate $dP(t)/dt,$ it is assumed that its size should not exceed a certain maximum rate $dP_{max}(t)/dt$. This maximum speed $dP_{max}(t)/dt$ is estimated by the empirical formula based on the data from cultivation experiments:

$$dP_{max}(t)/dt = \frac{x(t)}{Age(t)^2} \cdot k_{dP}; \qquad (7)$$

where the realistically possible maximum rate of synthesis of the product concentration of a young bacterial culture $dP_{max}(t)/dt$ is determined by the coefficient $k_{dP}$. According to the data of cultivation experiments, the value of this coefficient is $k_{dP} = 0.15$.

In the fourth step of the method, according to the formed $\mu$-profile of the bacterial specific growth rate, the automatic controller of the bioreactor controls the flow of the nutrient substrate, thus maintaining the real specific growth rate defined by the $\mu$-profile in the $\mu$ bioreactor. The automatic nutrient flow controller works in a known way, evaluating the real specific growth rate $\mu$ in the bioreactor according to the real-time measured oxygen utilization rate data [6] (Galvanauskas, Vytautas; Simutis, Rimvydas; Levišauskas, Donatas; Urniežius, Renaldas. Practical Solutions for Specific Growth Rate Control Systems in Industrial Bioreactors // Processes. Basel: MDPI. ISSN 2227-9717. 2019, vol. 7, iss. 10, art. No. 693, p. 1-1.

The scheme of the control system of the cultivation process of *E.coli,* applying the method of maximizing the output of the target product described in the invention, is shown in Fig. 2.

During the cultivation of *E.coli,* the biomass concentration $x(t)$ and the average age of the bacterial population *Age(t)* are evaluated in real time according to the gas flow and composition leaving the bioreactor. Until the moment of induction $t_{induction}$, the flow of nutrient substrate is supplied according to a profile pre-planned by the operator. After the process operator has performed the dosing of the inducer (IPTG) and initiated the synthesis of the target product, at the moment of induction $t_{induction}$, the probable maximum concentration of the target product $P_{max}$ at the end of cultivation $t_{end}$ is determined and the procedure for maximizing the synthesis of the target product is initiated. According to this procedure, taking into account the limitations of the process (maximum specific growth rate, maximum release rate of the target product), the specific growth profile $\mu(i*\mathrm{d}t),i=0..n$ is optimized (the most suitable is formed), which with the help of the specific growth rate $\mu$ controller controls the flow of nutrient substrate in the post-induction period [$t_{induction}$; $t_{end}$]. The controller of the specific growth rate $\mu$ functions according to the known control method, according to the data of the oxygen consumption rate **[6]** (Galvanauskas, Vytautas; Simutis, Rimvydas; Levišauskas, Donatas; Urniežius, Renaldas. *Practical Solutions for Specific Growth Rate Control Systems in Industrial Bioreactors* // Processes. Basel: MDPI. ISSN 2227-9717. 2019, vol. 7, iss. 10, art. No. 693, p. 1-13.).

The developed *E.coli* cultivation management method enables maximizing the concentration of the target product in semi-periodic recombinant *E.coli* cultivation processes, optimally controlling the specific bacterial growth rate $\mu$ in the post-induction period. The method can be effectively used in cultivation processes and industrial bioreactors of biopharmaceutical target products of various purposes.

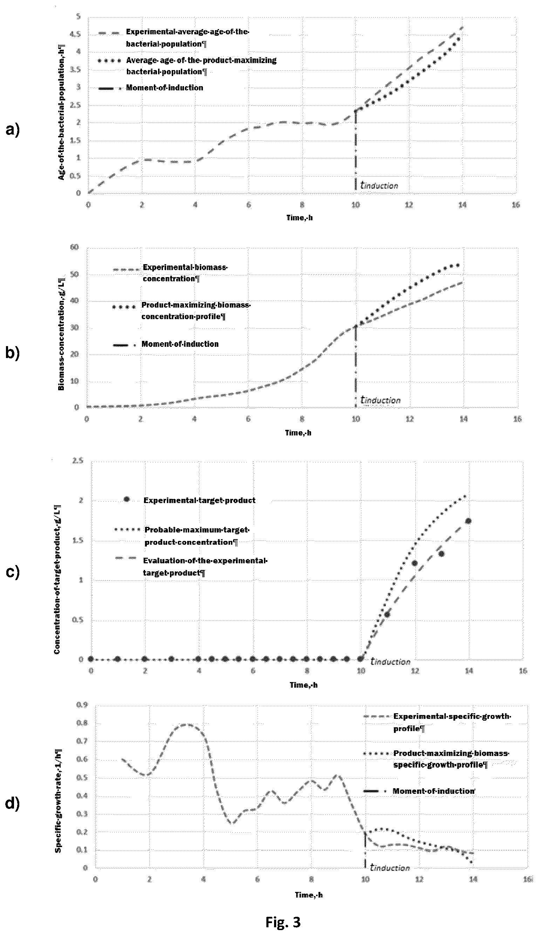

**Experimental results.** The method of maximizing the target product disclosed in the invention was verified analytically in the cultivation processes of recombinant *E.coli.* A typical result of the implementation of this method for one experiment in a real *E.coli* biomass cultivation and target product synthesis experiment is presented in Fig. 3. The figure shows the *E.coli* biomass concentration $x(t)$ (Fig. 3 a), the average age of the bacterial population *Age(t)* (Fig. 3 b), the variation of the specific growth rate $\mu$ (Fig. 3 c) and the concentration of the target product growth $P(t)$ graphs. In the bioprocess, the development of a specific growth rate profile is carried out:

- According to the operator's prior plan - in the pre-induction (E.coli biomass cultivation) phase, and
- According to the method of this invention - in the post-induction (target product synthesis) phase in order to maximize the target product synthesis yield at the end of cultivation.

After the operator selects the moment of induction $t_{induction}$, the biomass concentration $x(t)$ and the average age of bacteria *Age(t)* at the moment of initiation of induction $t_{induction}$ can have different values. Optimization of the synthesis of the target product and maximization of the yield $P(t_{end})$ can, in fact, be performed for any values of $x(t_{induction})$ and *Age(t_{induction})* at the time of initiation of induction $t_{induction}$.

After initiation of induction, from the moment $t_{induction}$ (Fig. 3: 10th hour), the specific growth rate $\mu$ is found based on the maximum of the function $P(t)$ of the two parameters $x(t)$ and *Age(t),* and ensuring that the limitations are not exceeded according to formulas (6) and (7). After induction, biomass growth slows down and the average age of bacteria increases. Cultivation characteristics achieved in reality will differ from those predicted by specific growth profiles, these differences can be seen in the dotted curves in Fig. 3 (mean population age), Fig. 3b (biomass concentration), Fig. 3c (maximum expected product concentration), Fig. 3d (specific growth rate).

In this analysis, applying the developed maximization method, the maximum expected concentration of the target product is on average 12% higher than it would have been if the synthesis process had not been optimized according to the mentioned two essential parameters: *E.coli* biomass concentration $x(t)$ and average age of bacteria *Age(t).*

LIST OF LITERATURE

**1.** US Patent US5595905A, 1997. Process Control System for Fed-Batch Fermentation Using a Computer to Predict Nutrient Consumption.

**2.** US Patent US6284453B1, 2001. Method for Controlling Fermentation Growth and Metabolism.

**3.** LT Patent LT6395B, Automatic Control System for Growth Rate of Microorganisms, 2015.

**4.** Gnoth, Stefan; Simutis, Rimvydas; Lübbert, Andreas. Selective Expression of the Soluble Product Fraction in Escherichia Coli Cultures Employed in Recombinant Protein Production Processes // Applied Microbiology and Biotechnology. New York: Springer. ISSN 0175-7598. 2010, Vol. 87, iss. 6, p. 2047-2058

**5.** Survyla, A., Urniezius, R. & Simutis, R. Viable Cell Estimation of Mammalian Cells Using Off-Gas-Based Oxygen Uptake Rate and Aging-Specific Functional. Talanta 254, 124121, 2023

**6.** Galvanauskas, Vytautas; Simutis, Rimvydas; Levišauskas, Donatas; Urniežius, Renaldas. Practical Solutions for Specific Growth Rate Control Systems in Industrial Bioreactors // Processes. Basel: MDPI. ISSN 2227-9717. 2019, vol. 7, iss. 10, art. no. 693, p. 1-13.)

**7.** Survyla, A., Levisauskas, D., Urniezius, R. & Simutis, R. An Oxygen-Uptake-Rate-Based Estimator of the Specific Growth Rate in Escherichia Coli BI21 Strains Cultivation Processes. Computational and Structural Biotechnology Journal 19, 5856-5863 (2021).

**8.** Schaepe, S., Kuprijanov, A., Simutis, R. & Lübbert, A. Avoiding Overfeeding in High Cell Density Fed-batch Cultures of E. coli During the Production of Heterologous Proteins. Journal of Biotechnology 192, 146-153 (2014).

**Claims**

1. A method to maximize the synthesis of the target product in semi-batch cultivation processes of recombinant *Ecoli,* comprising the following sequence of steps:

   a) In the first step (1.1), the *E.coli* biomass concentration $x(t)$ is estimated and the average age of the population *Age(t)* is determined during the cultivation period until the moment of induction $t_{induction}$;
   b) In the second step (1.2), at the moment of induction $t_{induction}$, according to the measured biomass concentration $x(t_{induction})$ and the estimated average age *Age(t_{induction})*, and applying the experimentally identified dependence of the product synthesis rate $dP(t)/dt$ on the biomass concentration $x(t)$ and age *Age(t),* the maximum concentration of the target product $P_{max}$ at the end of cultivation at the time $t_{end}$ is estimated;
   c) In the third step (1.3), according to the experimentally identified dependence of the product synthesis rate $dP(t)/dt$, the product concentration function $P(t)$ in the post-induction period is estimated $[t_{induction}; t_{end}]$, and by the iterative optimization procedure a profile $\mu(t)$ of the specific growth rate of *E.coli* is created, which corresponds to the expected maximum concentration of the product $P(t)$ at the end of cultivation at the moment $t_{end}$.
   d) In the fourth step (1.4), the nutrient substrate flow is supplied to the bioreactor,controlled by an automatic controller according to the optimized specific growth rate profile $\mu(t), t=[t_{induction}; t_{end}]$.

2. The method according to claim 1, **characterized in that** in the first step (a), the *E.coli* biomass concentration $x(t)$ is estimated in real time, by indirect measurement according to the rate of oxygen consumption in the reactor.

3. The method according to claim 1 or 2, **characterized in that** the average age *Age(t)* of the *E.coli* population is estimated based on the estimated *E.coli* biomass concentration profile $x(t)$.

4. The method according to claim 3, **characterized in that** *Age(t)* is calculated according to the formula:

$$Age(t_i) = \frac{t_i \cdot x(t_i) - \sum_{j=1}^{i} t_j \cdot \Delta x_{j,j-1}}{x(t_i)}.$$

5. The method according to claim 1, **characterized in that** in the second step (b) the expected maximum concentration limit of the target product $P_{max}$ at the end of the cultivation is estimated according to the formula

$$P_{max} = \frac{x(t_{induction})}{Age(t_{induction})^2} * Y_{ap} \; [g/L] \, ,$$

where $Y_{ap}$ represents the experimentally estimated yield specific coefficient of the cultivation process characterizing the capacity of the process to synthesize the target product.

6. The method according to claim 5, **characterized in that** the yield specific coefficient $Y_{ap}$ is equal to 0.5.

7. The method according to claim 1, 5 and 6, **characterized in that** in the third step (c), the function of the concentration of the synthesized product $P(t)$ is calculated in the period $t=[t_{induction}; t_{end}]$ according to the formula:

$$P(t_i) = P(t_{i-1}) + \frac{P_{max} - P(t_{i-1})}{Age(t_i)} \Delta t_i,$$

- The *E.coli* biomass concentration function *x(t)* is calculated according to the formula:

$$x(t_i) = x(t_{induction}) \cdot e^{\sum_{k=t_{induction}}^{i} \mu(t_k) \cdot \Delta t_k}$$

- The average age function *Age(t)* is calculated, and
- The optimized function $\mu(t)$-profile of the *E.coli* specific growth rate is formed by the iterative optimization procedure, which tends to enable the expected maximum product concentration *P(t)* at the end of the cultivation period.

8.  The method according to claim 7, **characterized in that** the iterative optimization procedure and the *E.coli* specific growth rate function is carried out, and $\mu(t)$-profile is optimized in a discrete time df.

9.  The method according to claim 8, **characterized in that** the size of the discrete time interval d*t* is selected from 0.1 to 1 hour.

10. The method according to any of the claims 7 to 9, **characterized in that** in the optimization iterative procedure, the $\mu$ *(t)*-profile of the optimized function of the specific growth rate of *E. coli* is formed by varying the values of the $\mu(t)$-profile in iterations, from 0 to 0.8, with a discrete step 0.01.

11. The method according to any of the claims 7 to 10, **characterized in that** in the iterative optimization procedure, the $\mu$ *(t)*-profile of the specific growth rate of *E.coli* is formed by applying limitations on the increase of $\mu(i{*}dt)$ values according to the Monod formula:

$$\mu_{Uplimit}(t) = \mu_{max} \cdot \frac{kx}{(kx + x(t))} \cdot \frac{kage}{(kage + Age(t)^2)} \cdot \frac{kp}{(kp + P(t)^2)},$$

where $\mu_{max}$ represents the maximum specific growth rate of *E.coli* culture, *kx* represents the biomass inhibition coefficient, *kage* represents the method age inhibition coefficient, and *kp* represents the product inhibition coefficient.

12. The method according to claim 11, **characterized in that** the limiting parameters in the Monod formula have the following values: $\mu_{max}$ = 1.1 [1/hr], *kx* = 80 [g/L], *kage* = 3.5 [hr], and *kp* = 20 [g/L].

13. The method according to any one of claims 7 to 12, **characterized in that** the optimization iterative procedure limits the maximum rate of synthesis of the target product according to the formula d*P$_{max}$*(*t*)/d*t*

$$\mathrm{d}P_{max}(t)/\mathrm{d}t = \frac{x(t)}{Age(t)^2} \cdot k_{dP};$$

where the parameter k$_{dP}$ represents the rate-limiting coefficient of synthesis of the target product concentration of a young bacterial culture.

14. The method according to claim 13, **characterized in that the** coefficient $Y_{ap}$ is equal to 0.15.

15. The method according to anyone of claims 1 to 14, **characterized in that** the method is designed to maximize the synthesis of the target product in industrial and automated *E.coli* cultivation processes and bioreactors.

**1.1**

1. Evaluation of biomass concentration x(t) and average age of bacteria Age(t) during *E.coli* cultivation process

**1.2**

2. Determination of the expected maximum concentration of the target product at the end of cultivation, at the moment of induction $t_{induction}$

**1.3**

3. An iterative procedure for target product maximization: optimization of a specific growth profile

**1.4**

4. Use of the specific growth rate profile in a bioreactor automatic controller to adjust the nutrient substrate flow

**2**

**3**

Bioreactor automatic controller

**4**

**5**

**6**

Nutrient substrate flow

Bioreactor and *E. coli* cultivation process therein

**Fig. 1**

**Fig. 2**

Fig. 3

Fig. 4

| | Europäisches Patentamt European Patent Office Office européen des brevets | **EUROPEAN SEARCH REPORT** | Application Number EP 23 19 1755 |
|---|---|---|---|

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | EP 3 385 366 A1 (SIEMENS AG [DE]) 10 October 2018 (2018-10-10) * paragraphs [0001], [0009], [0011] – [0016], [0019], [0034], [0036] * ----- | 1-15 | INV. C12M1/34 C12M1/36 C12N1/20 |
| Y,D | US 6 284 453 B1 (SIANO STEVEN ANTHONY [US]) 4 September 2001 (2001-09-04) * columns 1-3 * * column 16, lines 25-28 * * columns 17-18 * * column 19, lines 6-11 * ----- | 1-15 | |
| Y,D | LT 6 395 B (KAUNO TECH UNIV [LT]) 12 June 2017 (2017-06-12) * paragraphs [0002], [0004], [0006] – [0009], [0011], [0017], [0025] * ----- | 1-15 | |
| A | DE 601 19 883 T2 (BECTON DICKINSON CO [US]) 21 December 2006 (2006-12-21) * paragraphs [0007], [0013] * ----- | 1-15 | |
| A | DE 697 27 069 T2 (PHOTONICS BIOSYSTEMS REDMOND [US]) 4 November 2004 (2004-11-04) * paragraphs [0001], [0002], [0004] * ----- | 1 | TECHNICAL FIELDS SEARCHED (IPC) C12M C12R C12N |
| A | DE 36 88 534 T2 (HITACHI LTD [JP]) 20 January 1994 (1994-01-20) * pages 1,2,6 * ----- | 1 | |
| A,D | US 5 595 905 A (BISHOP BRUCE F [US] ET AL) 21 January 1997 (1997-01-21) * the whole document * ----- | 1 | |

The present search report has been drawn up for all claims

| Place of search | Date of completion of the search | Examiner |
|---|---|---|
| Berlin | 12 March 2024 | Böhm, Ingo |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

 

& : member of the same patent family, corresponding document

EPO FORM 1503 03.82 (P04C01)

# EP 4 481 031 A1

## ANNEX TO THE EUROPEAN SEARCH REPORT
## ON EUROPEAN PATENT APPLICATION NO.

EP 23 19 1755

This annex lists the patent family members relating to the patent documents cited in the above-mentioned European search report.
The members are as contained in the European Patent Office EDP file on
The European Patent Office is in no way liable for these particulars which are merely given for the purpose of information.

12-03-2024

| Patent document cited in search report | | Publication date | Patent family member(s) | | Publication date |
|---|---|---|---|---|---|
| EP 3385366 | A1 | 10-10-2018 | CN | 110462018 A | 15-11-2019 |
| | | | EP | 3385366 A1 | 10-10-2018 |
| | | | US | 2020190460 A1 | 18-06-2020 |
| | | | WO | 2018185052 A1 | 11-10-2018 |
| US 6284453 | B1 | 04-09-2001 | NONE | | |
| LT 6395 | B | 12-06-2017 | NONE | | |
| DE 60119883 | T2 | 21-12-2006 | DE | 60119883 T2 | 21-12-2006 |
| | | | EP | 1234872 A2 | 28-08-2002 |
| | | | EP | 1724335 A1 | 22-11-2006 |
| | | | US | 2003111607 A1 | 19-06-2003 |
| DE 69727069 | T2 | 04-11-2004 | AT | E257178 T1 | 15-01-2004 |
| | | | AU | 5079698 A | 05-05-1998 |
| | | | DE | 69727069 T2 | 04-11-2004 |
| | | | EP | 0934428 A1 | 11-08-1999 |
| | | | US | 8173438 B1 | 08-05-2012 |
| | | | WO | 9815645 A1 | 16-04-1998 |
| DE 3688534 | T2 | 20-01-1994 | CN | 86101928 A | 24-09-1986 |
| | | | DE | 3688534 T2 | 20-01-1994 |
| | | | EP | 0196061 A2 | 01-10-1986 |
| | | | JP | H078231 B2 | 01-02-1995 |
| | | | JP | S61219379 A | 29-09-1986 |
| | | | KR | 860007378 A | 10-10-1986 |
| | | | US | 4891310 A | 02-01-1990 |
| US 5595905 | A | 21-01-1997 | NONE | | |

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 5595905 A
- US 6284453 B1

- LT 6395 B

**Non-patent literature cited in the description**

- Selective Expression of the Soluble Product Fraction in Escherichia Coli Cultures Employed in Recombinant Protein Production Processes. **GNOTH, STEFAN** ; **SIMUTIS, RIMVYDAS** ; **LÜBBERT, ANDREAS**. Applied Microbiology and Biotechnology. Springer, 2010, vol. 87, 2047-2058
- **SURVYLA, A.** ; **URNIEZIUS, R.** ; **SIMUTIS, R.** Viable Cell Estimation of Mammalian Cells Using Off-Gas-Based Oxygen Uptake Rate and Aging-Specific Functional.. *Talanta*, 2023, vol. 254, 124121
- **SURVYLA, A.** ; **URNIEZIUS, R.** ; **SIMUTIS, R.** Viable Cell Estimation of Mammalian Cells Using Off-Gas-Based Oxygen Uptake Rate and Aging-Specific Functional. *Talanta*, 2023, vol. 254, 124121
- **SURVYLA, A.** ; **LEVISAUSKAS, D.** ; **URNIEZIUS, R** ; **SIMUTIS, R.** An Oxygen-Uptake-Rate-Based Estimator of the Specific Growth Rate in Escherichia Coli BI21 Strains Cultivation Processes. *Computational and Structural Biotechnology Journal*, 2021, vol. 19, 5856-5863

- **SCHAEPE, S.** ; **KUPRIJANOV, A.** ; **SIMUTIS, R.** ; **LÜBBERT, A**. Avoiding Overfeeding in High Cell Density Fed-Batch Cultures of E. Coli During the Production of Heterologous Proteins.. *Journal of Biotechnology*, 2014, vol. 192, 146-153
- Selective Expression of the Soluble Product Fraction in Escherichia Coli Cultures Employed in Recombinant Protein Production Processes. **GNOTH, STEFAN** ; **SIMUTIS, RIMVYDAS** ; **LÜBBERT, ANDREAS**. Applied Microbiology and Biotechnology.. Springer, 2010, vol. 87, 2047-2058
- **SURVYLA, A.** ; **LEVISAUSKAS, D.** ; **URNIEZIUS, R.** ; **SIMUTIS, R.** An Oxygen-Uptake-Rate-Based Estimator of the Specific Growth Rate in Escherichia Coli BI21 Strains Cultivation Processes. *Computational and Structural Biotechnology Journal*, 2021, vol. 19, 5856-5863
- **SCHAEPE, S.** ; **KUPRIJANOV, A.** ; **SIMUTIS, R.** ; **LÜBBERT, A.** Avoiding Overfeeding in High Cell Density Fed-batch Cultures of E. coli During the Production of Heterologous Proteins.. *Journal of Biotechnology*, 2014, vol. 192, 146-153